(19) Europäisches Patentamt
European Patent Office
Office européen des brevets

(11) Veröffentlichungsnummer: **0 137 358**
**A2**

(12) # EUROPÄISCHE PATENTANMELDUNG

(21) Anmeldenummer: **84111052.1**

(22) Anmeldetag: **17.09.84**

(51) Int. Cl.⁴: **C 07 D 239/22**
A 01 N 43/54

(30) Priorität: **26.09.83 DE 3334799**

(43) Veröffentlichungstag der Anmeldung:
**17.04.85 Patentblatt 85/16**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Knops, Hans-Joachim, Dr.**
**Köpenicker Strasse 35**
**D-4019 Monheim(DE)**

(72) Erfinder: **Heinemann, Ulrich, Dr.**
**Feldstrasse 18**
**D-5653 Leichlingen 1(DE)**

(72) Erfinder: **Babczinski, Peter, Dr.**
**In der Lohrenbeck 11**
**D-5600 Wuppertal 1(DE)**

(72) Erfinder: **Eue, Ludwig, Dr.**
**Paul-Klee-Strasse 36**
**D-5090 Leverkusen 1(DE)**

(72) Erfinder: **Schmidt, Robert R. Dr.**
**Im Waldwinkel 110**
**D-5060 Bergisch-Gladbach 2(DE)**

(54) **1,3-Diaryl-5-methylen-perhydropyrimidin-2-one.**

(57) Die Erfindung betrifft neue 1,3-Diaryl-5-methylen-perhydropyrimidin-2-one der allgemeinen Formel (I),

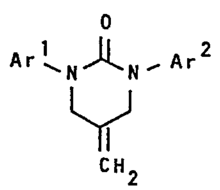

(I)

in welcher

$Ar^1$ und $Ar^2$ unabhängig voneinander für gegebenenfalls substituiertes Aryl stehen,

ein Verfahren zu ihrer Herstellung und ihre Verwendung als Herbizide.

EP 0 137 358 A2

BAYER AKTIENGESELLSCHAFT 5090 Leverkusen, Bayerwerk

Konzernverwaltung RP
Patentabteilung

Bi-klu

Ia

# 1,3-Diaryl-5-methylen-perhydropyrimidin-2-one

Die Erfindung betrifft neue 1,3-Diaryl-5-methylen-perhydropyrimidin-2-one, ein Verfahren zu ihrer Herstellung und ihre Verwendung als Herbizide.

Es ist bereits bekannt, daß manche cyclischen Harnstoffderivate wie beispielsweise das 1-Phenyl-3-(3-trifluormethylphenyl)-perhydropyrimidin-2-on oder das 5-Methyl-1-phenyl-3-(3-trifluormethylphenyl)-perhydropyrimidin-2-on herbizide Eigenschaften besitzen (vgl. EP-A-0 058 868 sowie EP-A-0 086 411).

Die Wirkung dieser Verbindungen ist jedoch, insbesondere bei niedrigen Aufwandmengen und -konzentrationen, nicht immer völlig zufriedenstellend.

Es wurden neue 1,3-Diaryl-5-methylenperhydropyrimidin-2-one der allgemeinen Formel (I),

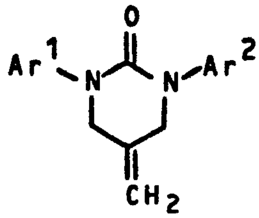

(I)

in welcher

$Ar^1$ und $Ar^2$ unabhängig voneinander für gegebenenfalls substituiertes Aryl stehen,

gefunden.

Weiterhin wurde gefunden, daß man die neuen 1,3-Diaryl-5-methylenperhydropyrimidin-2-one der allgemeinen Formel (I) erhält, wenn man N,N'-Diarylharnstoffe der allgemeinen Formel (II),

$$Ar^1\text{-NH-CO-NH-}Ar^2 \qquad (II)$$

in welcher

$Ar^1$ und $Ar^2$ unabhängig voneinander für gegebenenfalls substituiertes Aryl stehen,

mit 3-Chlor-2-chlormethylpropen der Formel (III)

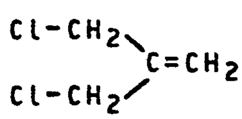

(III)

gegebenenfalls in Gegenwart eines Verdünnungsmittels, gegebenenfalls in Gegenwart eines Säurebindemittels sowie gegebenenfalls in Gegenwart eines Katalysators umsetzt.

Schließlich wurde gefunden, daß die neuen 1,3-Diaryl-5-methylentetrahydropyrimidin-2-one der Formel (I) herbizide Eigenschaften besitzen.

Ueberraschenderweise zeigen die neuen 1,3-Diaryl-5-methylentetrahydropyrimidin-2-one der Formel (I) bessere herbizide Wirksamkeit als die aus dem Stand der Technik bekannten Verbindungen 1-Phenyl-3-(3-trifluormethylphenyl)-perhydropyrimidin-2-on bzw. 5-Methyl-1-phenyl-3-(3-trifluormethylphenyl)-perhydropyrimidin-2-on, welche chemisch und wirkungsmäßig naheliegende Verbindungen sind. Die erfindungsgemäßen Stoffe stellen somit einen überraschenden Fortschritt gegenüber dem Stand der Technik dar.

Die erfindungsgemäßen Stoffe sind durch die Formel (I) allgemein definiert. Bevorzugt sind Stoffe der Formel (I), bei denen

$Ar^1$ und $Ar^2$ unabhängig voneinander für α-Naphthyl oder für gegebenenfalls einfach oder mehrfach, gleich oder verschieden substituiertes Phenyl stehen, wobei als Substituenten vorzugsweise infrage kommen : Halogen (insbesondere Fluor, Chlor oder Brom), Alkyl, Alkoxy, Alkylthio, Alkylcarbonyl oder Alkoxycarbonyl mit jeweils 1 bis 4 Kohlenstoffatomen im Alkylteil, außerdem Nitro, Cyano, Halogenalkyl mit 1 bis 4 Kohlenstoffatomen und 1 bis 5 Halogenatomen (insbesondere Fluor, Chlor oder Brom) und Phenoxy.

Besonders bevorzugt sind Verbindungen der Formel (I), bei denen

$Ar^1$ und $Ar^2$ unabhängig voneinander für α-Naphthyl oder für gegebenenfalls ein- bis dreifach, gleich oder verschieden substituiertes Phenyl stehen, wobei als Substituenten besonders bevorzugt sind : Fluor, Chlor, Brom, Methyl,Ethyl, i-Propyl, Methoxy, Ethoxy, Isopropyloxy, Methylthio, Phenoxy, Nitro, Cyano, Acetyl, Methoxycarbonyl, Ethoxycarbonyl und Trifluormethyl.

Im einzelnen seien außer den bei den Herstellungsbeispielen genannten Verbindungen die folgenden Stoffe der allgemeinen Formel (I) genannt :

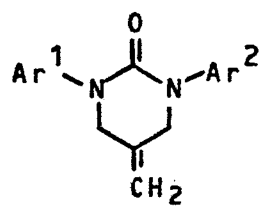

(I)

Tabelle 1: erfindungsgemäße Verbindungen der Formel (I)

| Ar$^1$ | Ar$^2$ | Ar$^1$ | Ar$^2$ |
|---|---|---|---|
| 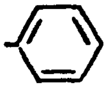 | 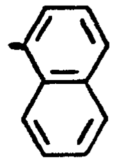 | 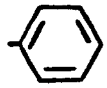 | 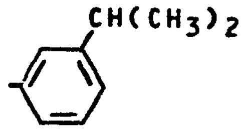 |
| 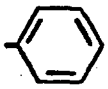 | 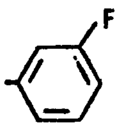 | 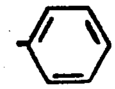 | 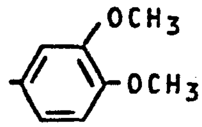 |
| 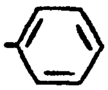 | 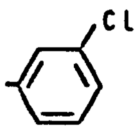 | 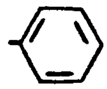 | 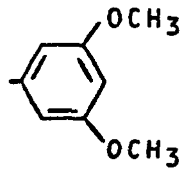 |
| 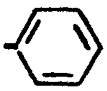 | 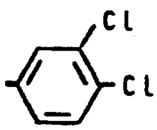 | 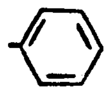 | 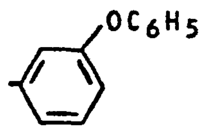 |
| 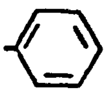 | 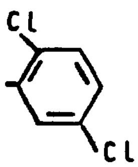 | 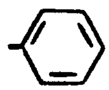 | 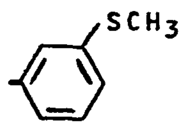 |
| 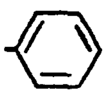 | 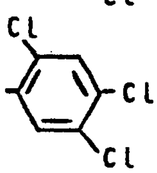 | 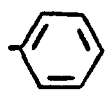 | 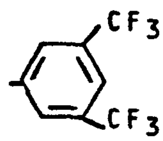 |
| 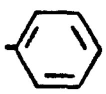 | 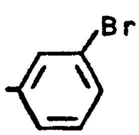 | 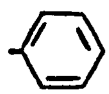 | 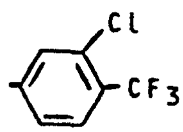 |
| 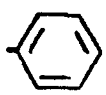 | 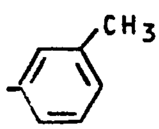 | 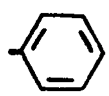 | 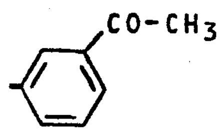 |
| 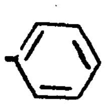 | 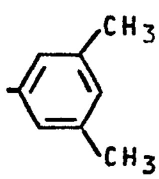 | 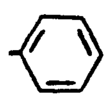 | 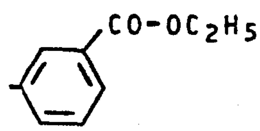 |
| | | 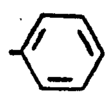 | 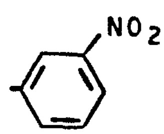 |

Fortsetzung Tabelle 1:

| $Ar^1$ | $Ar^2$ | $Ar^1$ | $Ar^2$ |
|---|---|---|---|
| 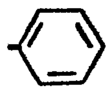 | 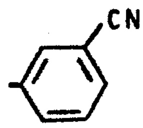 | 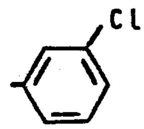 | 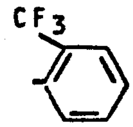 |
| 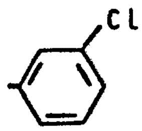 | 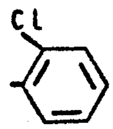 | 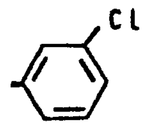 | 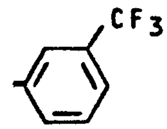 |
| 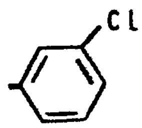 | 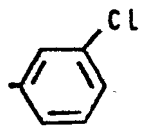 | 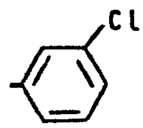 | 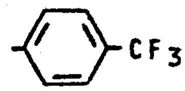 |
| 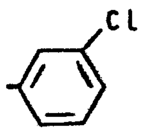 | 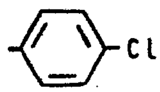 | 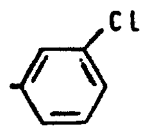 | 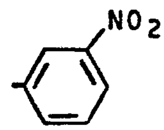 |
| 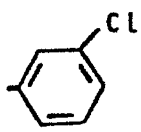 | 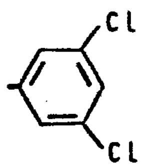 | 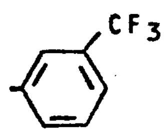 | 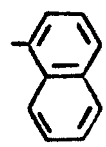 |
| 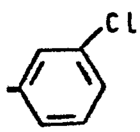 | 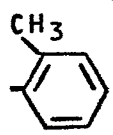 | 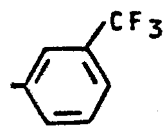 | 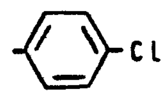 |
| 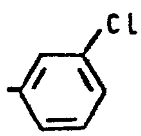 | 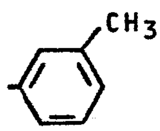 | 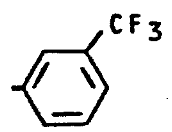 | 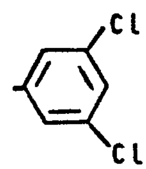 |
| 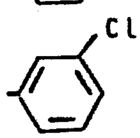 | 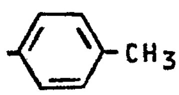 | 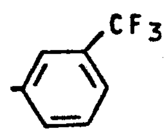 | 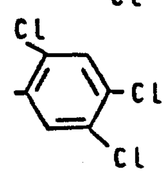 |
| 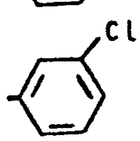 | 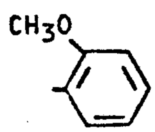 | 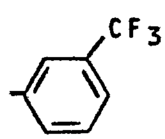 | 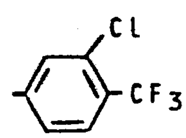 |
| 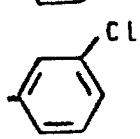 | 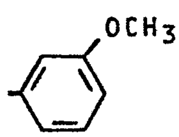 | | |
| 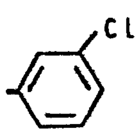 | 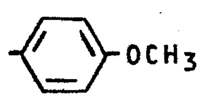 | | |

Fortsetzung Tabelle 1:

| Ar[1] | Ar[2] | Ar[1] | Ar[2] |
|---|---|---|---|
| 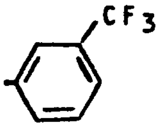 | 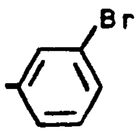 | 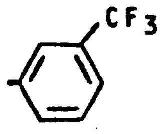 | 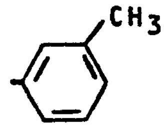 |
| 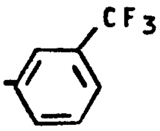 | 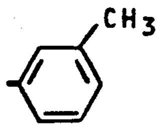 | 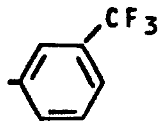 | 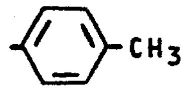 |
| 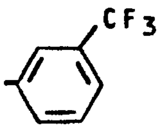 | 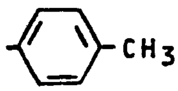 | 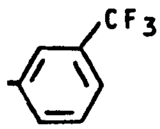 | 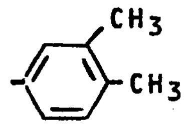 |
| 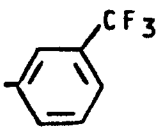 | 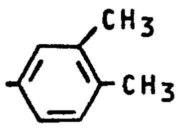 | 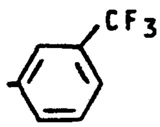 | 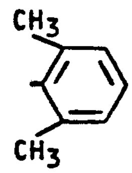 |
| 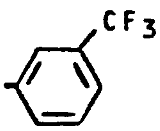 | 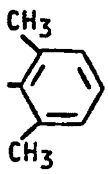 | 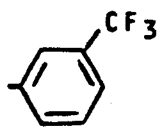 | 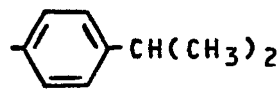 |
| 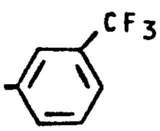 | 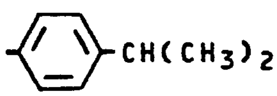 | 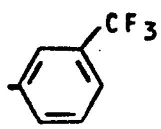 | 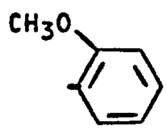 |
| 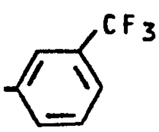 | 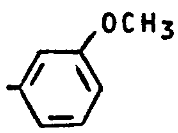 | 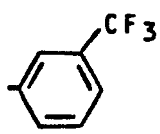 | 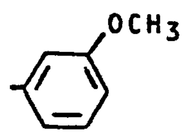 |
| 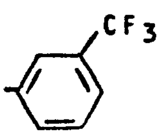 | 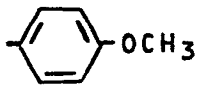 | 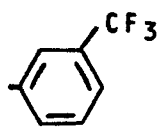 | 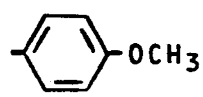 |
| 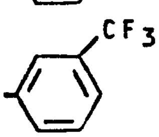 | 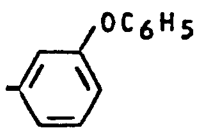 | 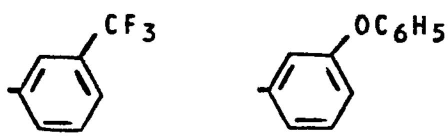 | |
| 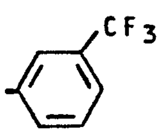 | 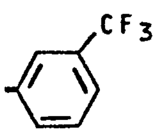 | 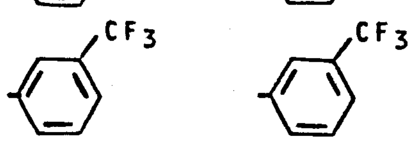 | |
| 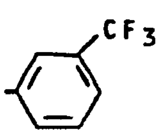 | 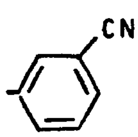 | | |

Fortsetzung Tabelle 1:

| $Ar^1$ | $Ar^2$ | $Ar^1$ | $Ar^2$ |
|---|---|---|---|
| 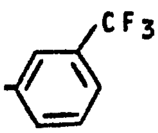 | 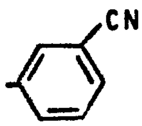 | 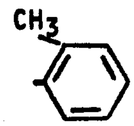 | 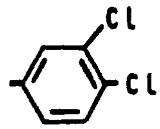 |
| 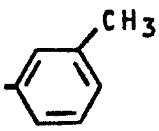 | 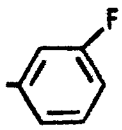 | 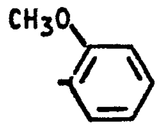 | 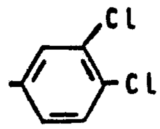 |
| 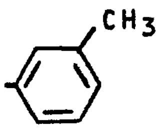 | 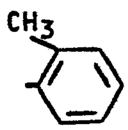 | | |
| 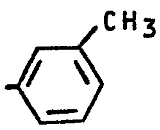 | 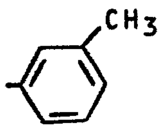 | | |
| 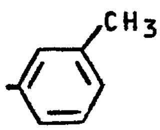 | 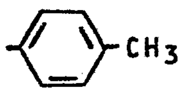 | | |
| 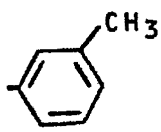 | 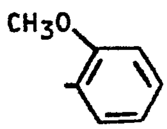 | | |
| 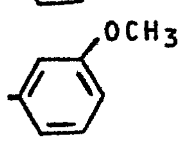 | 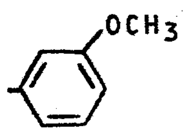 | | |
| 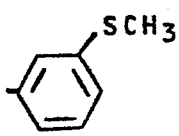 | 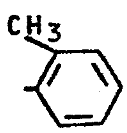 | | |
| 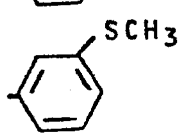 | 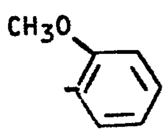 | | |
| 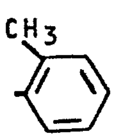 | 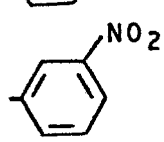 | | |

Verwendet man beispielsweise N-Phenyl-N'-(3-trifluormethylphenyl)-harnstoff und 3-Chlor-2-chlormethyl-propen als Ausgangsstoffe, so läßt sich der Ablauf des erfindungsgemäßen Verfahrens durch das folgende Formelschema darstellen :

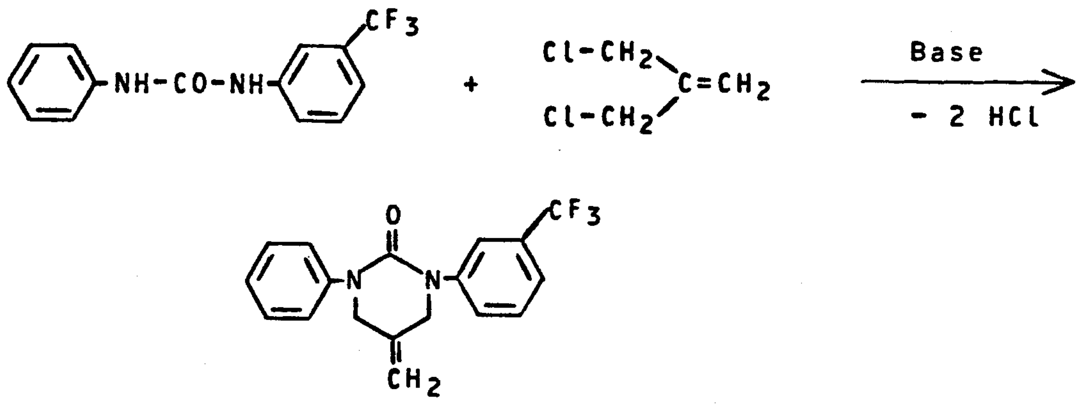


Die bei dem erfindungsgemäßen Verfahren als Ausgangsstoffe zu verwendenen N,N'-disubstituierten Harnstoffe sind durch die Formel (II) allgemein definiert. In dieser Formel stehen $Ar^1$ und $Ar^2$ vorzugsweise für diejenigen Reste, welche bei der Beschreibung der entsprechenden Reste in Formel (I) als bevorzugt angegeben wurden.

Die Verbindungen der Formel (II) sind bekannt [vgl. z.B. Ber.dtsch.Chem. <u>25</u>, 1366 (1892); J.Chem.Soc. <u>79</u>, 105 (1901)] oder können nach analogen Verfahren auf einfache Weise hergestellt werden, indem man z.B. aromatische Amine der Formel (IV),

$$Ar^1-NH_2 \qquad (IV)$$

in welcher

$Ar^1$ die in der Erfindungsdefinition angegebene Bedeutung hat,

mit Isocyanaten der Formel (V),

$$Ar^2-N=C=O \qquad (V)$$

in welcher

$Ar^2$ die in der Erfindungsdefinition angegebene Bedeutung hat,

gegebenenfalls in Gegenwart eines Verdünnungsmittels, wie z.B. Dichlormethan, bei Temperaturen zwischen -20°C und +100°C zur Reaktion bringt.

Das weiterhin für das erfindungsgemäße Verfahren als Ausgangsstoff benötigte 3-Chlor-2-chlormethylpropen ist durch die Formel (III) definiert. Die Verbindung der Formel (III) ist bekannt [vgl. D.E. Applequist/J.D. Roberts, J.Amer. Chem.Soc. 78, 4018 (1956)].

Das erfindungsgemäße Verfahren wird üblicherweise in Gegenwart eines geeigneten Verdünnungsmittels durchgeführt. Als solche kommen praktisch alle inerten organischen Lösungsmittel infrage. Vorzugsweise verwendet man aliphatische oder aromatische Kohlenwasserstoffe wie Benzol, Toluol, Xylol, Hexan oder Petrolether, halogenierte Kohlenwasserstoffe, wie Dichlormethan, Chloroform, Tetrachlorkohlenstoff, Chlorbenzol oder Dichlorbenzol oder dipolar aprotische Lösungsmittel wie z.B. Acetonitril

oder Propionitril, Dimethylformamid, N-Methylformanilid, Dimethylsulfoxid, Aceton, Butanon, Sulfolan und Hexamethylphosphorsäuretriamid.

Das erfindungsgemäße Verfahren wird üblicherweise in Gegenwart eines Säurebindemittels durchgeführt. Als solche kommen alle üblichen anorganischen und organischen Basen in Betracht.

Bevorzugt sind die stark basischen Alkalihydroxide, wie Natriumhydroxid oder Kaliumhydroxid oder auch Alkoholate, wie beispielsweise Kalium-t-butylat.

Das erfindungsgemäße Verfahren kann gegebenenfalls in Gegenwart eines Katalysators durchgeführt werden. Als Katalysatoren eignen sich besonders Alkalimetalliodide, vorzugsweise verwendet man Natrium- oder Kaliumiodid.

Die Reaktionstemperaturen können in einem breiten Bereich variiert werden. Allgemein arbeitet man bei Temperaturen zwischen 20°C und 160°C, insbesondere zwischen 50°C und 150°C.

Das erfindungsgemäße Verfahren wird im allgemeinen bei Normaldruck durchgeführt.

Zur Durchführung des erfindungsgemäßen Verfahrens setzt man pro Mol N,N'-disubstituierten Harnstoffes der Formel (II) 0,8 bis 1,0 Mol an 3-Chlor-2-chlormethylpropen der Formel (III) und 1,8 bis 2,4 Mol an Base ein. Vorzugsweise verwendet man äquimolare Mengen der Reaktionspartner der Formeln (II) und (III) und die doppelt molare Menge an Base.

In einer bevorzugten Ausführungsform löst man einen der Reaktionspartner - entweder den Harnstoff der Formel (II) oder das 3-Chlor-2-chlormethylpropen der Formel (III) - zusammen mit der doppelt molaren Menge an Base in dem entsprechenden Verdünnungsmittel und gibt danach die äquimolare Menge des anderen Reaktionspartners zu. Darauf wird die Reaktionsmischung auf die entsprechende Reaktionstemperatur gebracht. Die Aufarbeitung erfolgt nach üblichen Methoden. Eventuell auftretende Nebenprodukte oder Verunreinigungen trennt man gegebenenfalls durch fraktionierte Destillation oder durch chromatographische Reinigung ab.

Die erfindungsgemäße Umsetzung kann auch in einem Zweiphasensystem, wie beispielsweise wässrige Natron- oder Kalilauge/Toluol oder Methylenchlorid, gegebenenfalls unter Zusatz von 0,1 bis 1,0 Mol eines Phasentransferkatalysators, wie beispielsweise Ammonium- oder Phosphoniumverbindungen [beispielsweise seien Benzyl-dodecyl-dimethylammoniumchlorid oder Triethyl-benzylammoniumchlorid genannt] durchgeführt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Daneben zeigen die erfindungsgemäßen Wirkstoffe der Formel (I) in entsprechend niedrigeren Aufwandmengen auch eine fungizide Wirkung insbesondere gegen den Erreger des Apfelschorfes (Venturia inaequalis).

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

0137353

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,1 und 15 kg Wirkstoff pro Hektar Bodenfläche, vorzugsweise zwischen 0,5 und 10 kg pro ha.

Die Herstellung und die Verwendung der erfindungsgemäßen Wirkstoffe geht aus den nachfolgenden Beispielen hervor.

## Herstellungsbeispiele :

### Beispiel 1 :

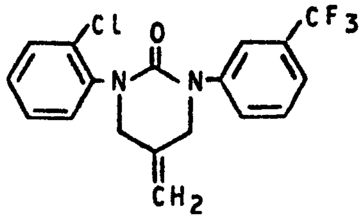

Zu 10 g (0,032 Mol) 2-Chlor-3'-trifluormethyl-diphenylharnstoff in 40 ml wasserfreiem Dimethylsulfoxid gibt man bei Raumtemperatur unter Rühren zunächst 4,0 g (0,071 Mol) Kaliumhydroxid und anschließend tropfenweise 4,25 g (0,034 Mol) 3-Chlor-2-chlormethylpropen. Nach beendeter Zugabe erhitzt man die Reaktionsmischung für 20 Minuten auf 70°C und rührt dann weitere 2 Stunden bei 50°C bis 60°C. Zur abgekühlten Reaktionsmischung gibt man 100 ml Wasser und extrahiert mehrfach mit Dichlormethan; die vereinigten organischen Phasen werden mit Wasser gewaschen, über Natriumsulfat getrocknet und im Vakuum eingeengt. Der Rückstand wird mittels Kugelrohrdestillation von flüchtigen Anteilen befreit und anschließend säulenchromatographisch gereinigt (Kieselgel/Laufmittel : Chloroform).

Man erhält 2,8 g (22,5 % der Theorie) an 1-(2-Chlorphenyl)-3-(3-trifluormethylphenyl)-5-methylenperhydropyrimidin-2-on vom Brechungsindex $n_D^{20}$ 1.5718.

0137358

Beispiel 2 :

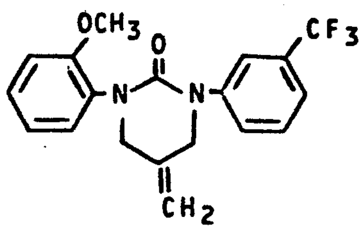

Zu einer Lösung von 10 g (0,032 Mol) 2-Methoxy-3'-trifluormethyl-diphenylharnstoff und 4,7 g (0,032 Mol) 3-Chlor-2-chlormethylpropen in 50 ml trockenem Toluol gibt man bei Raumtemperatur unter Rühren 7,18 g (0,064 Mol) Kalium-t-butylat. Nach beendeter Zugabe rührt man eine Stunde bei Raumtemperatur und weitere 3 Stunden unter Rückfluß des Lösungsmittels. Zur Aufarbeitung gibt man 100 ml Wasser zu der erhaltenen Reaktionsmischung, trennt die organische Phase ab, wäscht sie zweimal mit Wasser, trocknet über Natriumsulfat und entfernt das Lösungsmittel im Vakuum. Nach säulenchromatographischer Reinigung des öligen Rückstandes (Kieselgel/Laufmittel : Chloroform) erhält man 4,0 g (34,5 % der Theorie) an 1-(2-Methoxyphenyl)-3-(3-trifluormethylphenyl)-5-methylen-perhydropyrimidin-2-on vom Brechungsindex $n_D^{20}$ 1.5508.

In entsprechender Weise und gemäß den allgemeinen Herstellungsangaben erhält man die folgenden Verbindungen der allgemeinen Formel (I) :

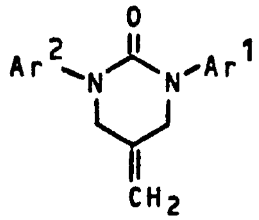

(I)

Tabelle 2:

| Beisp. Nr. | $Ar^1$ | $Ar^2$ | Schmelzpunkt (°C) oder Brechungsindex ($n_D^{20}$) |
|---|---|---|---|
| 3 | 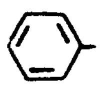 | 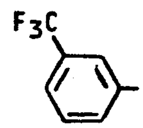 | Fp. 89°C |
| 4 | 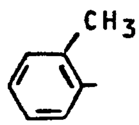 | 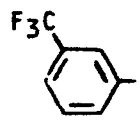 | Fp. 83,5°C |
| 5 | 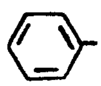 | 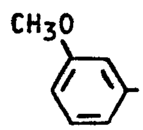 | $n_D^{20}$ 1.6052 |
| 6 | 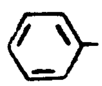 | 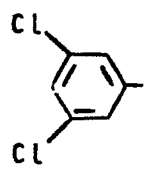 | Fp. 148°C |
| 7 | 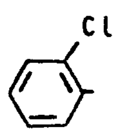 | 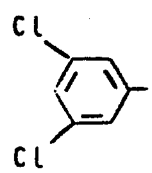 | $n_D^{20}$ 1.610 |

Fortsetzung Tabelle 2:

| Beisp. Nr. | $Ar^1$ | $Ar^2$ | Schmelzpunkt (°C) oder Brechungsindex ($n_D^{20}$) |
|---|---|---|---|
| 8 | 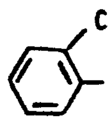 | 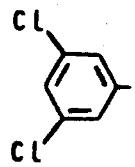 | Fp. 103°C |
| 9 | 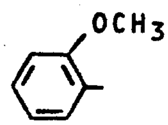 | 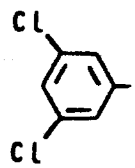 | |
| 10 | 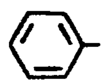 | 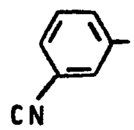 | |
| 11 | 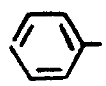 | 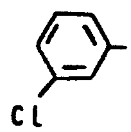 | $n_D^{20}$ 1.55 |
| 12 | 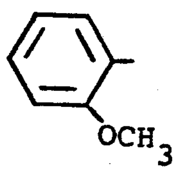 | 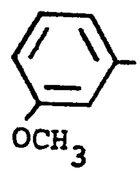 | $n_D^{25}$: 1.5792 |

Anwendungsbeispiele :

In den folgenden Anwendungsbeispielen werden die nachstehend angegebenen Verbindungen als Vergleichssubstanzen herangezogen :

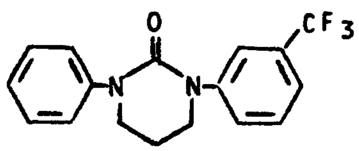

(A)

1-Phenyl-3-(3-trifluormethylphenyl)-perhydropyrimidin-2-on (bekannt aus EP-A- 0 058 868);

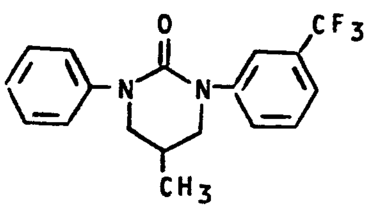

(B)

5-Methyl-1-phenyl-3-(3-trifluormethylphenyl)-perhydropyrimidin-2-on (bekannt aus EP-A- 0 086 411).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen z.B. die folgenden Verbindungen gemäß den Herstellungsbeispielen eine ausgezeichnete Wirksamkeit: (2), (3).

Patentansprüche

1) 1,3-Diaryl-5-methylen-perhydropyrimidin-2-one der allgemeinen Formel (I),

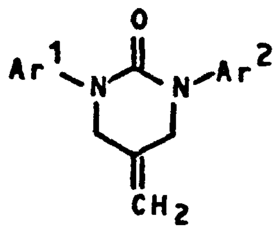

(I)

in welcher
$Ar^1$ und $Ar^2$ unabhängig voneinander für gegebenenfalls substituiertes Aryl stehen.

2) 1,3-Diaryl-5-methylen-perhydropyrimidin-2-one der allgemeinen Formel (I) gemäß Anspruch 1, dadurch gekennzeichnet, daß

$Ar^1$ und $Ar^2$ unabhängig voneinander für $\alpha$-Naphthyl oder für gegebenenfalls einfach oder mehrfach, gleich oder verschieden durch Halogen, Alkyl, Alkoxy, Alkylthio, Alkylcarbonyl oder Alkoxycarbonyl mit jeweils 1-4 C-Atomen im Alkylteil, durch Nitro, Cyano, Halogenalkyl mit 1-4 C-Atomen und 1-5 Halogenatomen oder durch Phenoxy substituiertes Phenyl stehen.

3) 1,3-Diaryl-5-methylen-perhydropyrimidin-2-one der allgemeinen Formel (I) gemäß Anspruch 1, dadurch gekennzeichnet, daß

$Ar^1$ und $Ar^2$ unabhängig voneinander für α-Naphthyl oder für gegebenenfalls ein- bis dreifach, gleich oder verschieden durch Fluor, Chlor, Brom, Methyl, Ethyl, i-Propyl, Methoxy, Ethoxy, Isopropyloxy, Methylthio, Phenoxy, Nitro, Cyano, Acetyl, Methoxycarbonyl, Ethoxycarbonyl oder Trifluormethyl substituiertes Phenyl stehen.

4) 1-(2-Methoxyphenyl)-3-(3-trifluormethylphenyl)-5-methylen-perhydropyrimidin-2-on gemäß Anspruch 1.

5) 1-Phenyl-3-(3-trifluormethyl-phenyl)-5-methylen-perhydropyrimidin-2-on gemäß Anspruch 1.

6) Verfahren zur Herstellung von 1,3-Diaryl-5-methylen-perhydropyrimidin-2-onen der Formel (I) gemäß Anspruch 1, dadurch gekennzeichnet, daß man N,N'-Diarylharnstoffe der allgemeinen Formel (II),

$$Ar^1\text{-NH-CO-NH-}Ar^2 \qquad (II)$$

in welcher

$Ar^1$ und $Ar^2$ unabhängig voneinander für gegebenenfalls substituiertes Aryl stehen,

mit 3-Chlor-2-chlormethylpropen der Formel (III)

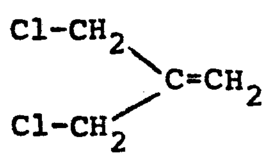

(III)

gegebenenfalls in Gegenwart eines Verdünnungsmittels, gegebenenfalls in Gegenwart eines Säurebindemittels sowie gegebenenfalls in Gegenwart eines Katalysators umsetzt.

7) Herbizide Mittel, gekennzeichnet durch einen Gehalt an mindestens einem 1,3-Diaryl-5-methylen-perhydropyrimidin-2-on der Formel (I) gemäß Anspruch 1.

8) Verwendung von 1,3-Diaryl-5-methylenperhydropyrimidin-2-onen der Formel (I) gemäß Anspruch 1 zur Bekämpfung von unerwünschtem Pflanzenwachstum.

9) Verfahren zur Herstellung von herbiziden Mitteln, dadurch gekennzeichnet, daß man 1,3-Diaryl-5-methylenperhydropyrimidin-2-one der Formel (I) gemäß Anspruch 1 mit Streckmitteln und/oder oberflächenaktiven Mitteln vermischt.